(19) Europäisches Patentamt
European Patent Office
Office européen des brevets

(11) **EP 3 316 768 B1**

(12) **EUROPÄISCHE PATENTSCHRIFT**

(45) Veröffentlichungstag und Bekanntmachung des Hinweises auf die Patenterteilung:
**27.05.2020 Patentblatt 2020/22**

(21) Anmeldenummer: **15733749.4**

(22) Anmeldetag: **02.07.2015**

(51) Int Cl.:
*A61B 5/021* (2006.01)   *A61B 5/024* (2006.01)
*A61B 5/0245* (2006.01)   *A61B 5/0432* (2006.01)
*A61B 5/00* (2006.01)   *A61B 18/14* (2006.01)
*A61B 18/00* (2006.01)

(86) Internationale Anmeldenummer:
**PCT/EP2015/065129**

(87) Internationale Veröffentlichungsnummer:
**WO 2017/001023 (05.01.2017 Gazette 2017/01)**

(54) **VORRICHTUNG UND VERFAHREN ZUR ÜBERWACHUNG UND MESSUNG DES AUTOREGULATIONSMECHANISMUS DES BLUTDRUCKS BEI EINEM LEBEWESEN**

DEVICE AND METHOD FOR MONITORING AND MEASURING THE AUTOREGULATION MECHANISM OF THE BLOOD PRESSURE IN A LIVING BEING

DISPOSITIF ET PROCÉDÉ POUR LA SURVEILLANCE ET LA MESURE DU MÉCANISME D'AUTORÉGULATION DE LA PRESSION SANGUINE CHEZ UN ÊTRE VIVANT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**09.05.2018 Patentblatt 2018/19**

(73) Patentinhaber: **Schwarzer Cardiotek GmbH 74076 Heilbronn (DE)**

(72) Erfinder:
• **BIENEK, Carsten**
**74196 Neuenstadt a. K. (DE)**
• **SPÄTH, Michael**
**70619 Stuttgart (DE)**
• **VOLLMER, Cornelius Fritz**
**74182 Obersulm (DE)**

(74) Vertreter: **Prinz & Partner mbB Patent- und Rechtsanwälte Rundfunkplatz 2 80335 München (DE)**

(56) Entgegenhaltungen:
EP-A1- 2 364 643    WO-A2-2005/043628
JP-A- 2013 202 123    US-B1- 7 163 512

## Beschreibung

GEBIET DER ERFINDUNG

Die Erfindung betrifft eine Vorrichtung und ein Verfahren zur Überwachung und/ oder Messung des Autoregulationsmechanismus des Blutdrucks bei Lebewesen.

HINTERGRUND

Die Autoregulationsmechanismen des Blutdrucks beispielsweise beim Menschen stellen sicher, dass das Herz-Kreislaufsystem auf plötzliche Blutdruckschwankungen (Lageveränderung, Verletzung, usw.) entsprechend reagiert, damit körperliche Schäden durch zu hohen oder zu niedrigen Blutdruck vermieden werden.

Man kann durch die Messung dieser Autoregulation Rückschlüsse auf die autonome Steuerung der Herzkreislauftätigkeit ziehen. Anwendungen liegen beispielsweise in der Risikobewertung von Herz - Kreislauferkrankungen. Speziell das Risiko eines plötzlichen Herztodes scheint mit der vagalen Steuerung des Herz-Kreislaufsystems direkt zusammenzuhängen.

Eine Methode zur Messung der Autoregulation ist die Messung der baroreflektorischen Systemantwort. Im Baroreflex-Regelmechanismus wird der Blutdruck mittels Barorezeptoren im Aortenbogen sowie den Karotiden verfolgt und bei steigendem oder fallendem Blutdruck Signale an das vegetative Nervensystem gegeben. Bei steigendem Blutdruck wird das sympathische Nervensystem gedämpft und das parasympathische aktiviert. Dies führt unter anderem zu einer Senkung der Herzfrequenz und Dilatation des Gefäßsystems. Dies sind Maßnahmen, die kurzfristig den Blutdruck stabilisieren können. Da diese Regelungen in beide Richtungen arbeiten, findet ein ständiges Nivellieren um einen bestimmten Blutdruck statt. Dieser auch in Ruhe stattfindende Regelungsprozess manifestiert sich in langwelligen (ca. 0,1 Hz) Blutdruck und Herzraten-Schwankungen.

Zur Messung des Baroreflexes wird herkömmlich ein Verfahren verwendet, bei dem der Blutdruck durch externe Maßnahmen erhöht oder erniedrigt wird (Kipptisch, pharmakologisch, externer Druck auf Halsschlagadern, usw.) und dabei kontinuierlich der Blutdruck und die Herzrate gemessen werden. Man trägt dann die Herzrate über dem Blutdruck auf (jeweils ein Wert für jeden Herzschlag) und interpoliert eine Gerade durch entsprechend aktivierten Bereich. Die Steigung ergibt die Baroreflexsensitivität (BRS) in ms/mmHg.

Dieses Verfahren ist insofern problematisch, da die Messung meist invasiv (Injektion von Medikamentenbolus) ist und durch die Medikamente oder die Blutdruck-Aktivierung selbst weitere Regelparameter verändert werden können.

Alternativ bietet sich die Messung der langwelligen Ruheregulationen an. Dabei wird mittels kontinuierlichem EKG und kontinuierlicher Blutdruckmessung über einen Zeitraum bis zu mehreren Stunden ein Datensatz zur weiteren Analyse gewonnen. Der Patient befindet sich in dieser Zeit in Ruhe, so dass nur die autoregulativen Mechanismen sichtbar seien sollen. Aus den zeitlichen Verläufen von Blutdruck und Herzschlagintervallwerten für jeden Herzschlag lassen sich entsprechende Korrelationen zwischen Blutdruck und Herzratenvariabilität ableiten.

Ein bekanntes Verfahren hierzu ist die sogenannte Sequenzmethode. Dabei werden in der Blutdruckreihe Sequenzen von mindestens drei aufeinander folgenden steigenden (oder fallenden) Blutdruckwerten gesucht. Zu diesen Sequenzen sucht man in den Herzratenwerten entsprechend fallende (steigende) Sequenzen, entweder beim gleichen Herzschlag startend oder um einen oder mehrere Herzschläge verschoben, je nach zugrundegelegtem Modell. Aus diesen gehörigen Sequenzen werden wiederum Steigungen berechnet und gemittelt und diese als BRS Werte angegeben.

Das Problem dieser Messung liegt in der Ermittlung dieser Sequenzen. In Ruhe sind die zugrundeliegenden Regelmechanismen schwach ausgeprägt und die Blutdruckänderungen gering. Dadurch können sie leicht durch Rauschen überlagert sein und werden dadurch nicht erkannt. Dies führt zu langen Messzeiten oder statistisch nicht aussagekräftigen Mittelwerten.

Es existieren weitere Korrelationsmethoden im Frequenzbereich der Messwerte oder basierend auf anderen Korrelationsparametern zwischen Blutdruckkurve und Herzrate, die aber wesentlich komplizierter in der Berechnung und Interpretation sind.

Nachteilig an den bekannten korrelationsbasierten Verfahren ist, dass die zugrundeliegenden Regelmechanismen zwar eine relativ konstante Wellenlänge in Ruhe haben, jedoch durch physiologische Vorgänge keine kohärente Phase aufweisen. Durch Bewegung, Atmung, Husten oder ähnliche Vorgänge können diese Regelungen kurze "Aussetzer" von einigen wenigen Herzschlägen haben, was eine normale frequenzbasierte Korrelationsanalyse erschwert.

Alle vorgenannten Verfahren haben gemeinsam, dass sie eine kontinuierliche Blutdruckmessung benötigen. Die kontinuierliche Herzratenmessung erfolgt unproblematisch über eine normale EKG-Ableitung und die Bestimmung der R-Zacken im EKG. Das Herzschlagintervall wird über den zeitlichen Abstand der R-Zacken definiert.

Die kontinuierliche Blutdruckmessung erfolgt nicht-invasiv, typischerweise durch Fingermanschetten, bei denen durch einen Regelkreislauf der Manschettendruck genau auf dem Pulsdruck im Finger gehalten wird. Gemessen wird dies durch eine IR Diode, welche die pulsatile Volumenänderung aufzeichnet. Diese Methode hat den Nachteil, dass sie aufgrund der Abschnürung am Finger unangenehm für den Patienten ist und damit auch nicht über längere Zeiträume angewendet werden kann. Weiterhin ist die Handhabung der Fingermanschette durch Druckschläuche und zusätzliche Höhenkorrektursensoren umständlich.

Weiterhin ist zur Bestimmung des Blutdrucks aus dem Manschettendruck eine regelmäßige Kalibrierung erforderlich. Bei dieser wird während mehrerer Sekunden die Manschetten intervallartig bis zu einem Maximalwert aufgepumpt und wieder abgelassen, so dass eine klassische oszillatorische Blutdruckmessung zur Eichung erfolgen kann. Für diesen Zeitraum existieren keine kontinuierlichen Blutdruckdaten, was bedeutet, dass diese nicht zur Messung beitragen können. Im Extremfall kann durch das starke Aufpumpen der Manschette sogar der Blutdruck selbst beeinflusst werden.

Aus dem Artikel "Bivariate phase-rectified signal averaging - a novel technique for cross-correlation analysis in noisy nonstationary signals", Journal of Electrocardiology 42 (2009), 602-606, A. Bauer et al, ist das bivariate phasenrektifizierte Mittelwertverfahren (bivariate phase-rectified signal averaging = BPRSA) bekannt. Einerseits beschreibt dieses Verfahren eine sogenannte phasenrektifizierte Mittelung, bei der (physiologische) Signale deren Phase unterbrochen sein kann durch entsprechende synchronisierte Mittelung der Frequenzanalyse wieder zugänglich gemacht werden. Weiterhin wird das Verhältnis zweier verschiedener synchron aufgezeichneter Biosignale betrachtet, um entsprechende Korrelationen zu beschreiben. Auch das hier beschriebene Verfahren weist jedoch die oben beschriebenen Nachteile bezüglich der Messung des Blutdrucks auf.

Verfahren und Systeme zur Messung eines Autoregulationsmechanismus des Blutdrucks sind aus der veröffentlichten Patentanmeldung WO 2005/043628 A2 und der Patentschrift US 7163512 B1 bekannt.

ZUSAMMENFASSUNG DER ERFINDUNG

Es ist eine Aufgabe der vorliegenden Erfindung eine einfache, schnelle und robuste Messung der baroreflektorischen Aktivität bereitzustellen, welche auf die herkömmliche Messung des Blutdrucks mittels einer Manschette verzichten kann.

Gemäß einem Aspekt der Erfindung wird ein Verfahren gemäß dem Gegenstand des Patentanspruchs 1 und ein System zur Überwachung und/ oder Messung eines Autoregulationsmechanismus des Blutdrucks bei einem Lebewesen unter Verwendung eines EKG-Signals gemäß dem Gegenstand des Patentanspruchs 6 bereitgestellt. Insbesondere dient das Verfahren zur Überwachung der baroreflektorischen Aktivität und/oder Sensitivität (BRS) des Lebewesens. Vorteilhaft umfasst das Verfahren die folgenden Schritte:

- Aufzeichnen des EKG-Signals des Lebewesens,
- Aufzeichnen einer Pulswellenkurve synchron (und gleichzeitig) zum Aufzeichnen des EKG Signals,
- Bestimmen von Herzratenintervallen aus dem EKG-Signal,
- Bestimmen einer Pulswellenlaufzeit zu jedem Herzratenintervall aus dem EKG Signal (bspw. der/den R-Zacke(n)) und der Pulswellenkurve,
- Bestimmen einer Mehrzahl signifikanter Änderungen der Pulswellenlaufzeiten nach einem vorgegebenen Kriterium,
- Auswahl jeweils eines zu jeder signifikanten Änderung der Pulswellenlaufzeiten zeitlich korrelierten Herzratenintervalls als Ankerpunkt,
- Auswählen einer bestimmten begrenzten Anzahl von zeitlich aufeinanderfolgenden Herzratenintervallen zeitlich vor und/oder nach jedem Ankerpunkt, um so eine begrenzte Sequenz von Herzratenintervallen zu jedem Ankerpunkt zu erzeugen,
- Mitteln mehrerer korrespondierender Herzratenintervalle jeder Sequenz eines jeweiligen Ankerpunktes über alle Sequenzen der Ankerpunkte,
- Bestimmen wenigstens eines Baroreflexsensitivitäts-Indikators basierend auf den gemittelten mehreren korrespondierenden Herzratenintervallen.

Die vorliegenden Erfindung stellt somit ein vorteilhaftes, kalibrationsloses, implizites Verfahren und System breit, die darauf basieren, dass lediglich Blutdruckänderungen von Herzschlag-zu-Herzschlag anhand der Änderung der Pulswellenlaufzeitintervalle von Herzschlag-zu-Herzschlag verwendet werden. Ferner werden vorteilhaft nur die Herzratenintervalle verwendet.

Generell wird ein EKG-Signals des Lebewesens aufgezeichnet und simultan dazu werden Pulswellenlaufzeiten aufgezeichnet. Die Pulswellenlaufzeit kann vorteilhaft mittels eines Pulsvolumendetektors zeitsynchron zur Aufzeichnung des EKG-Signals erfolgen. Ferner werden vorteilhaft die Herzratenintervalle aus dem EKG-Signal bestimmt. Änderungen der Pulswellenlaufzeit weisen dabei auf Änderungen des Blutdrucks hin. Für signifikante Änderungen des Blutdrucks basierend auf signifikanten Änderungen der Pulswellenlaufzeit zwischen zwei Herzschlägen können dann sogenannten Ankerpunkte in dem EKG Signal bzw. in den Herzratenintervallen bestimmt werden. Schließlich kann die bivariate phasenrektifizierte Mittelwertmethode (BPRSA) angewandt werden und es können Indikatorwerte (BRS) bestimmt werden.

Das vorliegende Verfahren und das System gemäß Aspekten und Ausführungsbeispielen der Erfindung greifen dabei auf Ankerpunkte im Verlauf bzw. einer Sequenz der Herzratenintervalle zurück. Ausgehend von diesen Ankerpunkten werden im Herzintervallverlauf Fenster links und rechts vom so definierten Herzschlag bzw. Herzratenintervall (Ankerpunkt) bestimmt. Es können beispielsweise 15 Herzratenintervalle vor und 15 Herzratenintervalle nach dem Ankerpunkt ausgewählt werden. Man erhält in diesem Beispiel ein Fenster von 31 Herzratenintervallen bzw. Herzschlägen mit einem phasenrektifizierten Signalverlauf. Zur Auswahl der Ankerpunkte kann auch eine bestimmte

Intervalländerung zwischen zwei R-Zacken aufeinanderfolgender Herzschläge dienen.

Um eine Korrelation zwischen Blutdruckänderung und Herzratenintervall zu bestimmen werden die Ankerpunkte aus einer spezifischen Blutdruckänderung bestimmt. Dahinter steht das Modell, dass beispielsweise ein Blutdruckanstieg sofort (mit ein bis zwei Herzschlägen Abstand) eine Änderung (Senkung) der Herzrate also einen Anstieg der Länge/Dauer des Herzratenintervalls bewirkt.

Im Mittelpunkt des Verfahrens steht also die Vorgehensweise, die Ankerpunkte nicht in der zu untersuchenden Messreihe zu suchen und zu setzen, sondern aus einer anderen Messreihe zu bestimmen, im vorliegenden Fall dem Blutdruck, dessen Änderung von Herzschlag zu Herzschlag mittels der Pulswellenlaufzeit bestimmt wird.

Beispielsweise wird immer dann wenn der Blutdruck von einem Schlag zum Nächsten ansteigt die Pulswellenzeit kleiner sein als im Herzschlag zuvor. Genau an diesem Herzschlag wird dann ein Ankerpunkt im Herzratenintervall gesetzt. Die gemittelte phasenrektifizierte Kurve gibt dann die gemittelte Änderung der Intervalldauer nach einem Blutdruckanstieg an. Aus der so erhaltenen Kurve lässt sich ablesen, wie stark die Herzrate fällt, wenn der Blutdruck steigt. Je nach Definition der Ankerpunkte lassen sich auch andere Korrelationen abbilden. Die komplizierten nichtlinearen Zusammenhänge (siehe auch die nachfolgenden Erläuterungen) zwischen dem absoluten Blutdruck und der Pulswellenlaufzeit können so außer Betracht bleiben.

Durch die Mittelung über sehr viele Ankerpunkte, dies können typischerweise 50% der Gesamtdaten sein, kann Rauschen effektiv unterdrückt werden und die Daten können effizient genutzt werden. Man erhält so eine Signalkurve, die die typische Blutdruckinduzierte Ruheschwingung der Herzratenänderung aufzeigt.

Die Dauer der Aufzeichnung des EKG-Signals und der Pulswellenkurve erfolgt vorteilhaft über 10 Minuten, es können aber auch Aufzeichnungen von bis zu mehreren Stunden erfolgen.

Der erfindungsgemäße Vorteil liegt in einem integrierten System, das ohne Manschetten-basierte Blutdruckbestimmung auskommt und in einem kombinierten, einfach zu bedienenden Gerät, welches die Messung nach vergleichsweise kurzer Messdauer ermöglicht. Anstatt der Manschetten-basierten Blutdruckmessung wird die Pulswellenlaufzeit für jeden Herzschlag verwendet. Die Pulswellenlaufzeit ist die Zeit, in der die von der Herzsystole erzeugte Pulswelle bis zu einem Pulswellendetektor z. B. am Finger gelaufen ist. Die Ausbreitungsgeschwindigkeit dieser Pulswelle hängt von verschiedenen Parametern ab. Unter anderem ist die Laufzeit abhängig von der Gefäßsteifigkeit, dem Abstand zum Herzen, dem Gefäßquerschnitt und auch vom systolischen Blutdruck beim Auswurf des Blutes in den Körperkreislauf. Während einer Messung in Ruhe können die physiologischen Parameter wie Abstand, Gefäßsteifigkeit, Viskosität des Blutes usw. als konstant angenommen werden, nur der Blutdruck bleibt als veränderliche Größe. Die genaue Abhängigkeit der Pulswellenlaufzeit vom Blutdruck ist komplex, nicht linear und von vielen schwer zu bestimmenden physiologischen Parametern abhängig. Allerdings gilt immer, dass bei ansonsten konstanten Parametern und steigendem Blutdruck die Pulswellen eine höhere Geschwindigkeit haben und umgekehrt. Damit verhält sich die Pulswellenlaufzeit genau umgekehrt zum Blutdruck was Herzschlag-zu-Herzschlag-Änderungen angeht. Gemäß Aspekten der vorliegenden Erfindung kann vorteilhaft auf die absolute Bestimmung des Blutdrucks verzichtet werden.

Somit lassen sich die Ankerpunkte mittels der bivariaten phasenrektifizierten Mittelwertmethode (BPRSA) auch mit diesen reinen Pulswellenlaufzeiten bestimmen. Die Absolutwerte des Blutdrucks werden nicht benötigt. Danach wird das Verfahren in herkömmlicher Weise angewendet und eine entsprechende BPRSA-Kurve berechnet. Aus den Werten um den Nullpunkt kann dann wiederum ein BRS-Index bestimmt werden.

Gemäß einem Aspekt der Erfindung, kann das Verfahren das Erkennen von R-Zacken in dem EKG Signal und Bestimmen der Pulswellenlaufzeiten unter Verwendung der R-Zacken umfassen. Es ist besonders vorteilhaft, dass lediglich die Detektion der R-Zacken im EKG Signal erforderlich ist. Dadurch können die Anforderungen an die Vorrichtung zur Aufzeichnung des EKG-Signals gering gehalten werden.

Zur Bestimmung der Pulswellenlaufzeit kann weiterhin die Ankunft der Pulswelle in der beobachteten Pulsvolumenänderung detektiert werden. Dazu kann der sogenannte Fußpunkt der Pulswelle bestimmt werden.

Zur Bestimmung des Fußpunktes kann die Signalkurve aus dem Detektor (bspw. Fingerclip oder Pflaster oder Klettband mit integrierten Sensoren etc.) nach Filterung und Glättung zweimal abgeleitet werden. Der Fußpunkt bestimmt sich dann aus dem Maximum der zweiten zeitlichen Ableitung in zeitlich sinnvollem Abstand zur R-Zacke und vor dem Maximum der ersten zeitlichen Ableitung.

Aufgrund von Rauschen in den Signalen und speziell den Ableitungen sind entsprechende Glättungsfilter anzupassen.

Alternativ kann durch mathematische Anpassung (least squares) einer passenden analytischen Funktion an den Signalabschnitt ein entsprechender Punkt des Anstiegs ermittelt werden. Neben den bekannten Anpassungen an kontinuierliche Polynome von mindestens Grad drei können auch zwei stückweise definierte Polynome unterschiedlichen Grades verwendet werden, bei denen entweder der Übergangspunkt zwischen den Polynomen (bzw. Polynomfunktionen) oder ein anderer charakteristischer Punkt den Fußpunkt repräsentiert. Durch entsprechende Minimierungs-Anpassungen an den ganzen Signalabschnitt sind diese Methoden robuster gegenüber Rauschen.

Ein System gemäß den Aspekten der Erfindung kann entsprechend eingerichtet sein, um eine oder mehrere der vorgenannten mathematischen Anpassungen vorzunehmen.

Das Verfahren kann ferner das Bestimmen der Herzratenintervalle basierend auf den R-Zacken in dem EKG-Signal umfassen. Dabei wird ein Herzratenintervall (RRi) als zeitlicher Abstand zweier aufeinanderfolgender R-Zacken zweier aufeinanderfolgender Herzschläge bestimmt.

Die Erfindung stellt ebenfalls ein vorteilhaftes System zur Überwachung und/ oder Diagnose eines Autoregulationsmechanismus des Blutdrucks bei einem Lebewesen basierend auf einem EKG-Signal gemäß dem Gegenstand des Patentanspruchs 6 bereit.

Das System kann eine Vorrichtung umfassen, welche eingerichtet ist um ein EKG-Signal des Lebewesens aufzuzeichnen und aus dem EKG-Signal Herzrateninterval des Lebewesens zu bestimmen. Ferner kann das System eine Vorrichtung umfassen, welche mindestens einen Pulswellensensor umfasst.

Das System kann vorteilhaft eingerichtet sein, um eine Mehrzahl (gleichartiger) signifikanter Änderungen des Blutdrucks anhand von Änderungen der Pulswellenlaufzeiten (bspw. beginnend mit der Herzsystole bis zum Pulswellensensor) zu bestimmen.

Ferner kann das System eingerichtet sein, um zu jedem Zeitpunkt einer signifikanten Änderung der Pulswellenlaufzeiten einen (zu den Änderungen des Blutdrucks) zeitlich korrelierten Ankerpunkt in dem Herzintervall-Signal zu bestimmen. Jeder Ankerpunkt ist einem Herzschlag aus dem Herzintervall-Signal zugeordnet. Die Selektion der Ankerpunkte findet nur noch bezüglich der einzelnen Herzschläge statt, welche dann vorteilhaft im Wesentlichen durch die R-Zacken gekennzeichnet werden.

Die Herzrateninterval können dann in zeitlich begrenzten Fenstern vor und/oder nach jedem Ankerpunkt (Herzschlag, R-Zacke des Herzschlags) aus dem EKG-Signal ausgewählt werden.

Das System kann schließlich eingerichtet sein, um die Herzrateninterval (dieser Fenster) über die Mehrzahl von Ankerpunkten zu mitteln.

Die Vorrichtung zur Bestimmung des Herzrateninterval kann ein EKG System sein, welches mindestens oder auch nur zwei, vorzugsweise drei, Elektroden umfasst. Da es lediglich auf die Detektion der R-Zacke ankommt, kann das EKG-System sehr einfach aufgebaut sein.

Insofern kann das EKG System lediglich zur Bestimmung der R-Zacken des EKG Signals ausgestaltet sein. Es kann ferner ausgestaltet sein, um die Herzrateninterval basierend auf den R-Zacken zu bestimmen.

Die Vorrichtung zur Bestimmung der Pulswellenlaufzeit kann vorteilhaft eine IR-Diode umfassen. Die IR-Diode kann ausgestaltet sein, um nur oder zumindest bei einer Wellenlänge von ca. 800 nm (bzw. im Bereich von 790 nm bis 810 nm), Licht auszusenden. Ein entsprechender IR-Sensor ist dann ebenfalls vorzusehen. Vorteilhaft können die IR-Diode und der entsprechende IR-Sensor in einem Fingerclip oder einem Band oder Pflaster am Finger untergebracht sein, so dass der Finger transmittierend oder reflektiv durch- bzw. beleuchtet wird. Dabei werden lediglich Volumenänderungen der Blutgefäße, bspw. am Finger detektiert. Die Änderung der Blutsauerstoffsättigung hat dann keinen Einfluss auf die Detektion der Pulswellenlaufzeiten.

Der Fingerclip kann vorteilhaft auch mindestens eine EKG-Elektrode umfassen. Mit anderen Worten sind dann die Sensoren und weiteren Komponenten zur Bestimmung der Pulswellenlaufzeit und zumindest teilweise des EKG-Signals in einem kompakten Sensor bzw. Fingerclip oder Klettband integriert/angeordnet.

Das System kann ferner einen ersten Vorverstärker, welcher ausgestaltet ist, das Signal des Pulswellensensors zu verstärken und/oder einen zweiten Vorverstärker, welcher ausgestaltet ist um Signale von EKG Elektroden zu verstärken und/oder einen Vorfilter zur Vorfilterung der Signale von dem Pulswellensensor und den EKG-Elektroden und/oder einen Analog-Digital-Wandler zur Analog-Digital Wandlung der Signale von dem Pulswellensensor und den EKG-Elektroden, umfassen.

Allgemein werden zunächst zeitsynchron das EKG-Signal und der Pulswellenverlauf aufgezeichnet. Das EKG-Signal wird durch mindestens eine entsprechende EKG-Ableitung kontinuierlich aufgenommen, welche mit den simultan dazu erfassten mit der jeweiligen Herzaktion korrespondieren Pulswellenverlauf in einem zeitsynchronen Zusammenhang stehen und welche durch mindestens einen Pulswellensensor an einem diesbezüglich arteriellen Ableitort (bspw. Finger) aufgenommen werden. Hieraus wird mindestens ein Kontrollparameter zum Beispiel in Form des Herzrateninterval bestimmt, welcher bspw. aus den jeweiligen RR-Intervallen (RRi) oder der jeweiligen Pulsfrequenz gewonnen wird. Ein weiterer Kontrollparameter hinsichtlich der Druck- bzw. Volumen-Regulation wird aus den gemessenen Pulswellenlaufzeit-Kurven bestimmt.

Vorteilhaft kann die zeitsynchrone und kontinuierliche Detektion von EKG-Signalen von mindestens einer Ableitposition nach einer der bekannten Ableitmethoden (Frank, Einthoven, Wilson und anderen) im Zusammenhang mit der Ableitung der jeweils mit dem Herzschlag korrespondierenden arteriellen Puls-Kurven von mindestens einem Ableitort erfolgen, wobei entsprechende Verfahren zur digitalen Datenerfassung und Messdatenaufbereitung Anwendung finden können.

Im vorliegenden Kontext wird das Signal, welches die Variation der Pulswellenlaufzeiten erfasst, auch als Trigger-Signal bezeichnet. Das Herzrateninterval-Signal wird als Zielsignal bezeichnet. Die Bestimmung der Ankerpunkte (auch als Zeitmarken zu bezeichnen) ist unter anderem eine besonders kritische Bedingung für die erfolgreiche Anwendbarkeit des Verfahrens.

Bei dem erfindungsgemäßen Verfahren können Schwellenwerte in Form von vorherbestimmten Signalamplituden oder vorherbestimmte Steigungen der Variation der Pulswellenlaufzeit als Trigger gewählt werden. Auch kann der Trigger aus Mittelung über mehrere, beispielsweise die letzten 3 oder 5, Herzschläge bestimmt werden. Die Wahl der entsprechend geeigneten Werte für einen Trigger sowie der Zeitfensters erfolgt durch vorangegangene empirische Untersuchungen. Zum Ausschluss von fehlerhaften Mehrfach-Triggerungen innerhalb derselben Herzaktion kann nach der erstmaligen Auslösung eines Triggers (bspw. Schwellenwertüberschreitung) der Vorgang für eine bestimmte Zeitdauer gesperrt werden.

Erfindungsgemäß wird demnach anhand der Variation der Pulswellenlaufzeit eine Herzschlag-zu-Herzschlag Änderung des Blutdrucks festgestellt. Vorliegend wird also lediglich die Änderung der Pulswellenlaufzeit von Herzschlag zu Herzschlag verwendet. Die absoluten Blutdruckwerte brauchen nicht bestimmt werden. Das stellt eine deutliche Verbesserung gegenüber Verfahren und Systemen dar, welche zusätzlich die absoluten Blutdruckwerte benötigen. Aus den Änderungen der Pulswellenlaufzeit von Herzschlag zu Herzschlag wird ein sogenannter Ankerpunkt innerhalb des Herzintervall-Signals ermittelt. So können aus der Zeitreihe bezüglich der Auftrittszeitpunkte aufeinanderfolgender R-Zacken die jeweiligen zeitlichen Abstände (Herzratenintervalle) ermittelt werden. Die zeitliche Entwicklung der hierbei auftretenden regulatorisch bedingten signifikanten Zeitdifferenzen aufeinanderfolgender R-R-Intervalle bzw. Herzratenintervalle kennzeichnet somit die Herzratenvariabilität.

Eine mögliche Verwendung des Verfahrens nach einem der Aspekte oder Ausführungsbeispiele der Erfindung und/oder eines Systems nach einem der Aspekte oder Ausführungsbeispiele der Erfindung liegt in der Bestimmung der Wahrscheinlichkeit des Therapieerfolgs des Lebewesens, insbesondere eines menschlichen Patienten, nach der renalen Denervation (RDN).

Die renale Denervation (renal denervation = RDN) ist die (katheterbasierte) Verödung von autonomen Nervenverbindungen von und zur Niere zur Behandlung bei therapieresistentem Bluthochdruck. Es hat sich jedoch herausgestellt, dass viele Patienten keine ausreichende Blutdrucksenkung in Reaktion auf diese Maßnahme zeigen. Das hier beschriebenen Verfahren und System ist geeignet mittels der BRS-Indikatoren Patienten zu selektieren, die auf RDN nicht ansprechen.

Der hierin beschriebene BRS-Indikator kann ebenfalls als ein Indikator für die Funktionsfähigkeit des autonomen kardialen Regelsystems genutzt werden und kann daher bei fortschreitender autonomer Neuropathie in Folge von Diabetes Mellitus abgeschwächt werden. Mit den Verfahren und Vorrichtungen gemäß der Erfindung kann ein kontinuierliches Monitoring des BRS über bspw. mehrere Monate erfolgen uns so kann der Fortschritt der autonomen Neuropathie gemessen werden bzw. bei einsetzender Therapie deren Erfolgsverlauf. Zu diesem

Zweck können auch andere abgeleitete Faktoren aus den Messwerten wie bspw. die Variation der Herzrate (Herzratenvariabilität) oder die Pulswellenlaufzeit als solches verwendet werden.

## KURZE BESCHREIBUNG DER FIGUREN

Weitere Vorteile und Merkmale der Erfindung ergeben sich aus der nachfolgenden Beschreibung der bevorzugten Ausführungsbeispiele der Erfindung anhand der beigefügten Figuren, wobei

    FIG. 1 eine vereinfachte Darstellung eines Systems gemäß einem Ausführungsbeispiel der Erfindung ist,

    FIG. 2 eine vereinfachte Darstellung des Verfahrens gemäß einem Ausführungsbespiel der Erfindung mittels eines Ablaufdiagramms ist,

    FIG. 3 Ausschnitte aus einem EKG-Signal und zugehörigem zeitsynchronem Pulswellenverlauf zeigt,

    FIG. 4 Ausschnitte aus einem EKG-Signal und zugehörigem zeitsynchronem Pulswellenverlauf zeigt,

    FIG. 5 ein Beispiel für die Ermittlung eines BRS-Indikators zeigt,

    FIG. 6 eine vereinfachte Darstellung eines Ausführungsbeispiels einer Vorrichtung bzw. eines Systems zeigt,

    FIG. 7 eine vereinfachte Darstellung eines Ausführungsbeispiels für die EKG-Elektroden und den Pulswellensensor ist, und

    FIG. 8 eine vereinfachte Darstellung eines Ausführungsbeispiels des Pulswellensensors ist.

## DETAILLIERTE BESCHREIBUNG DER AUSFÜHRUNGSBEISPIELE

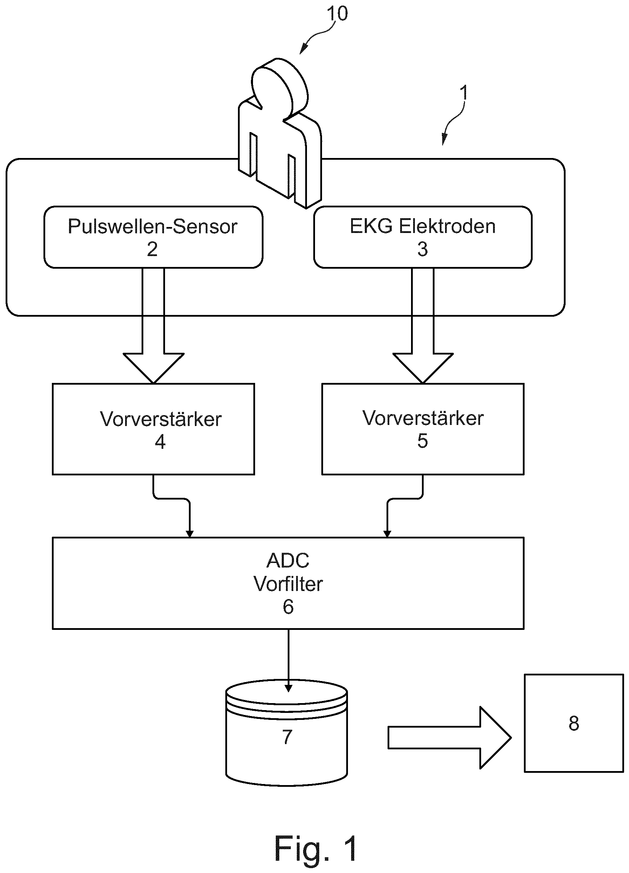

FIG. 1 ist eine vereinfachte Darstellung eines Systems 1 gemäß einem Ausführungsbeispiel der Erfindung. Das erfindungsgemäße System 1 umfasst ein Pulswellensensor 2, EKG-Elektroden 3, einen ersten Vorverstärker 4, einen zweiten Vorverstärker 5, einen analog Digitalwandler mit einem entsprechenden Vorfilter 6 und einen Speicher 7. Ferner liegt auch eine digitale Signalverarbeitungseinheit 7 beispielsweise in Form eines Computers 8 vor.

Der Pulswellensensor 2 kann als Fingerclip, längliches Band, insbesondere Klettband bzw. Band mit Klettverschluss oder Pflaster ausgestaltet sein. Dieser kann eine IR-Leuchtdiode und einen IR-Sensor umfassen. Vorteilhaft kann die IR-Leuchtdiode Licht im Bereich von 790 nm bis 810 nm, bzw. ca. 800 nm Wellenlänge aussenden. Der IR-Sensor kann dann ausgestaltet sein, um Licht dieser Wellenlänge quantitativ zu erfassen. Der Pulswellensensor 2 ist dann vorteilhaft als Pulsvolumensensor ausgestaltet, der lediglich die Volumenänderung am Finger erfasst.

Der Fingerclip kann vorteilhaft auch eine EKG-Elektrode umfassen. Dies kann die Handhabung deutlich erleichtern.

Die weitere Verarbeitung der erfassten, verstärkten und gegebenenfalls gefilterten sowie digitalisierten Signale, nämlich des EKG-Signals und des Pulswellensignals erfolgt dann bspw. in einem Computer 8 nach dem nachstehend beschriebenen Verfahren.

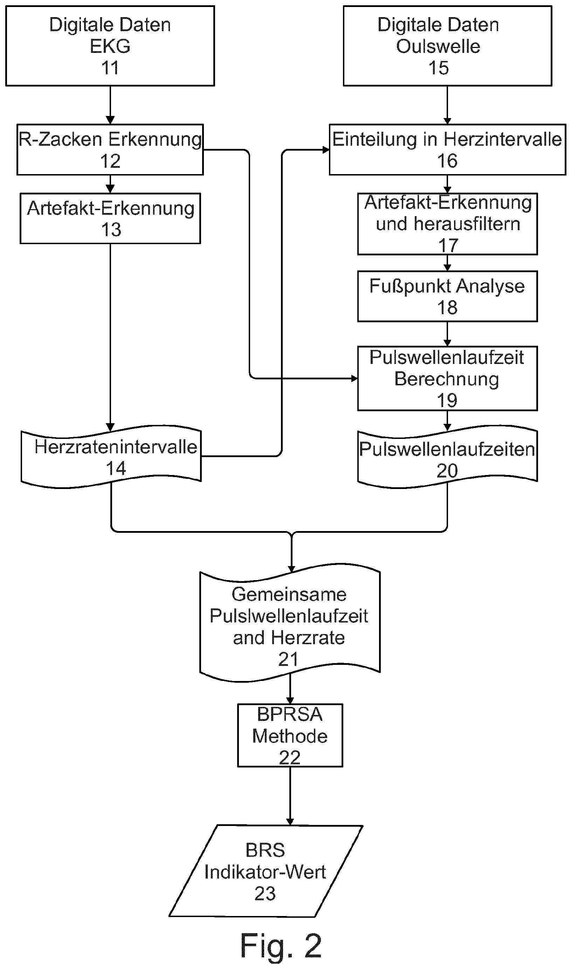

FIG. 2 ist eine vereinfachte Darstellung des Verfahrens gemäß einem Ausführungsbespiel der Erfindung mittels eines Ablaufdiagramms. Zunächst werden die digitalen Daten des EKGs in Schritt 11 erfasst. Simultan hierzu werden die digitalen Daten des Pulswellenverlaufs in Schritt 15 erfasst. Innerhalb der digitalen Daten des EKGs werden in Schritt 12 die R-Zacken erkannt. Vorteilhaft werden Zeitmarken für jede Position einer R-Zacke bzw. des Maximums der R-Zacke gesetzt. Ferner erfolgt eine Erkennung von Artefakten in den digitalen EKG-Daten in Schritt 13. Die R-Zacken-Erkennung in Schritt 12 wird gleichzeitig zur Berechnung der Pulswellenlaufzeit in Schritt 19 verwendet. Hier kann bspw. die R-Zacke als Startzeitpunkt und ein Fußpunkt des Pulswellenverlaufs am Pulswellensensor 2 als Endzeitpunkt für die Zeitdauer eine Pulswelle dienen. Aus den digitalen Daten des EKG werden nach der R-Zacken-Erkennung in Schritt 12 und der Artefakt-Erkennung in Schritt 13 die Herzrateintervalle (RRi) in Schritt 14 bestimmt. Diese Herzrateintervalle werden auf die digitalen Daten der Pulswelle aus Schritt 15 angewandt. Insbesondere werden sie zur Einteilung in Herzintervalle in Schritt 16 verwendet. Die in Schritt 16 eingeteilten Herzintervalle werden einer Artefakt-Erkennung und einer Filterung zur Unterdrückung der Artefakte in Schritt 17 unterzogen. Anschließend werden die Daten einer Fußpunktanalyse in Schritt 18 unterzogen. Anschließend wird in Schritt 19 unter Berücksichtigung der erkannten R-Zacken aus dem EKG-Signal die Pulswellenlaufzeit berechnet. Dies ergibt die aufbereiteten Pulswellenlaufzeiten, welche in Schritt 20 berücksichtigt werden. Aus den gespeicherten Herzrateintervallen in Schritt 14 und den Pulswellenlaufzeiten in Schritt 20 werden gemeinsame Pulswellenlaufzeiten und Herzraten in Schritt 21 bestimmt. Hierauf werden die Verfahren der bivariaten phasenrektifizierten Mittelwertmethode (BPRSA) in Schritt 22 angewandt. Schließlich ergibt sich hieraus ein BRS-Indikator in Schritt 23.

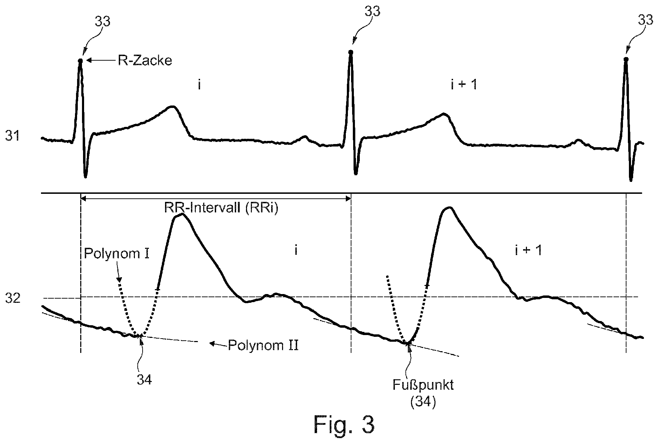

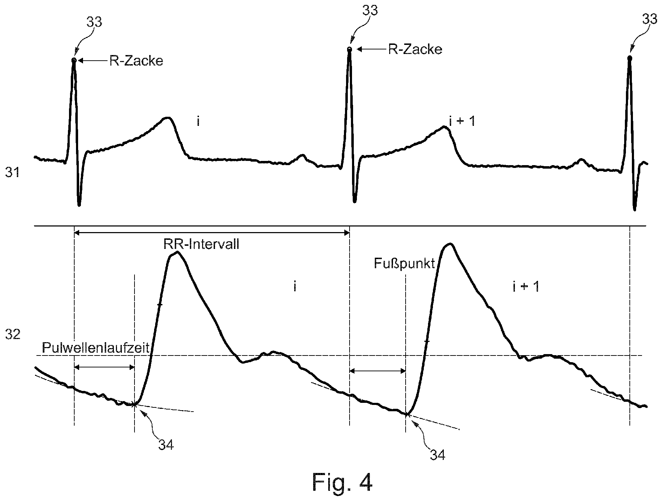

FIG. 3 und FIG. 4 zeigen jeweils einen Ausschnitt aus einem EKG-Signal 31 und einer zugehörigen zeitsynchron aufgenommen Pulswellenkurve 32 zur Erläuterung der Aspekte und Ausführungsbeispiele der Erfindung. Gemäß den Verfahren und Vorrichtungen der Erfindung wird aus dem EKG-Signal 31, wie in FIG. 3 dargestellt, zunächst das Maximum der R-Zacke 33 zu jedem Herzschlag bestimmt. Hieraus wird der zeitliche Abstand von einem Maximum 33 einer R-Zacke zu einem nächsten Maximum 33 des darauffolgenden Herzschlags als Herzrateintervall (RRi) bestimmt. Zeitlich synchron dazu wird die Pulswellenkurve 32 mittels des Pulswellensensors und der hierin beschriebenen Vorrichtungen aufgezeichnet. Unmittelbar nach dem Auftreten einer R-Zacke 33 fällt die Pulswellenkurve zunächst weiter ab bis sie einen Fußpunkt 34 erreicht, ab welchem sie im Wesentlichen wieder ansteigt.

Gemäß einem Ausführungsbeispiel der Erfindung wird nun, wie in FIG. 4 dargestellt, die Pulswellenlaufzeit jedes Herzschlags als zeitlicher Abstand der R-Zacke 33, genau genommen des Maximums der R-Zacke 33, zum Fußpunkt 34 bestimmt.

Zur Bestimmung des Fußpunktes 34 kann die Signalkurve aus dem Detektor (bspw. Fingerclip oder Pflaster) nach Filterung und Glättung zweimal abgeleitet werden. Der Fußpunkt 34 bestimmt sich aus dem Maximum der zweiten zeitlichen Ableitung in zeitlich sinnvollem Abstand zur R-Zacke und vor dem Maximum der ersten zeitlichen Ableitung. Der zeitlich sinnvolle Abstand kann bspw. aus physiologischen Erwägungen begrenzt werden auf:

a) länger als 150 ms, da eine gewisser Mindestabstand vom Finger zum Herzen (= Laufzeit) angenommen werden kann,

b) kürzer als das letzte oder mittlere Herzschlagintervall, da selbst bei langen Extremitäten eine so lange Laufzeit nicht vorkommen sollte, oder

c) alternativ kann auch eine feste obere Grenze von bspw. 600 ms gewählt werden.

Aufgrund von Rauschen in den Signalen und speziell den Ableitungen sind entsprechende Glättungsfilter einzusetzen bzw. anzupassen.

Alternativ kann durch mathematische Anpassung (Least Squares oder zu Deutsch: kleinstes Fehlerquadrat) einer passenden analytischen Funktion an den Signalabschnitt ein entsprechender Punkt des Anstiegs ermittelt werden. Neben den bekannten Anpassungen an kontinuierliche Polynome mindestens vom Grad drei können auch zwei stückweise definierte Polynome unterschiedlichen Grades verwendet werden, bei denen entweder der Übergangspunkt oder ein anderer charakteristischer Punkt den Fußpunkt 34 repräsentiert. Durch entsprechende Minimierungs-Anpassungen an den ganzen Signalabschnitt sind diese Methoden robuster gegenüber Rauschen.

Auf diese Art kann zu jedem Herzschlag eine zugehörige Pulswellenlaufzeit ermittelt werden. Verändert sich die Pulswellenlaufzeit signifikant, d. h. nach einem zuvor festgelegten Kriterium, dann wird der zugehörige Herzschlag bzw. das zugehörige Herzrateintervall (RRi) als Ankerpunkt ausgewählt. Hiervon ausgehend werden die zeitlich aufeinanderfolgenden davorliegenden und danach liegenden Herzrateintervalle in einer bestimmten Anzahl ausgewählt. Beispielsweise können vor dem als Ankerpunkt ausgewählten Herzratenintervall i eine bestimmte Anzahl von aufeinanderfolgenden, zeitlich vorher liegenden Herzrateninnervallen ausgewählt werden. Ebenso können zeitlich aufeinanderfolgende Herzrateninnervalle nach dem Herzrateninnervall i ausgewählt werden.

Als Kriterium zur Auswahl eines Herzratenintervalls als Ankerpunkt kommt beispielsweise in Betracht, wenn sich die Pulswellenlaufzeit von einem Herzschlag i zum nächsten Herzschlag i+1 vergrößert. Alternativ kann auch eine Differenz von mehr als bspw. eine oder mehr Millisekunden als Schwellwert angenommen werden, um Rauscheffekte auszufiltern. Es kommen aber auch andere Kriterien in Betracht, die auf eine signifikante Änderung des Blutdrucks hinweisen und empirisch bestimmt werden können.

Basierend auf dem gewählten, gleichen Kriterium werden in der Gesamtsequenz von Herzschlägen weitere Herzrateninnervalle (oder auch Herzschläge) als Ankerpunkte ausgewählt. Anschließend wird über alle Herzrateninnervalle mit dem gleichen Index, also den korrespondierenden Herzrateninnervallen, die zu den verschiedenen Ankerpunkten gehören, gemittelt.

Hat eine Sequenz von ausgewählten Herzrateninnervallen RR(k,i) bspw. eine Anzahl von 31 Herzschlägen, dann besitzt diese Sequenz 31 Herzrateninnervalle. 15 aufeinanderfolgende Herzrateninnervalle zeitlich vor dem Herzrateninnervall, das den Ankerpunkt darstellt, und 15 aufeinanderfolgende Herzrateninnervalle zeitlich nach dem Herzrateninnervall, das den Ankerpunkt darstellt.

Eine Sequenz mit der Nummer k hat demnach die Herzrateninnervalle RR(k,i) wobei i = -m...m (hier mit m=15) und k = 1..n. Die Zahl m ist eine ganze positive Zahl. Die Anzahl n der Sequenzen bestimmt sich aus der Anzahl der Ankerpunkte und kann zwischen 1 und einigen Hundert bis einigen Tausend liegen. Die Zahl n ist ebenfalls eine ganze positive Zahl. Gemittelt werden die Herzrateninnervalle RR(k,i) über die Variable k bei festem i. i=0 entspricht dabei dem Ankerpunkt bzw. dem Herzintervall des Ankerpunkts. Der Mittelwert $\overline{RR}_{(i)}$ wird demnach wie folgt berechnet:

$$\overline{RR}_{(i)} = \frac{1}{n} \sum_{k=1}^{n} RR_{(i,k)}$$

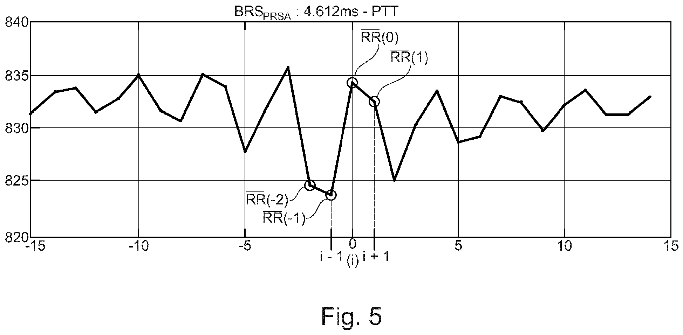

wobei k die Sequenz bezeichnet für die ein Ankerpunkt gewählt wurde und RR das Herzrateninnervall, also die Zeit von einer R-Zacke zu nächsten R-Zacke ist. Die $\overline{RR}_{(i)}$ können dann, wie in FIG. 5 beispielhaft für m=15 dargestellt, aufgetragen werden.

FIG. 5 zeigt das Ergebnis einer solchen beispielhaften Mittelung über 15 vor und 15 nach dem Ankerpunkt liegenden Herzrateninnervalle. Als typischer Wert kommen in etwa 50% aller Herzrateninnervalle als Ankerpunkt in Betracht. Je nach Art und Anzahl der Artefakte kann die Zahl jedoch auch geringer oder höher liegen. Der Mittelwert $\overline{RR}_{(0)}$ aller Herzrateninnervalle i=0 (also Ankerpunkte) für k von 1 bis n ist in der Mitte, also beim Index 0 aufgetragen. Daneben ist der gemittelte Wert $\overline{RR}_{(i+1)}$ der Pulswellenlaufzeiten aller Herzrateninnervalle für den Index i=1 (also dem Herzrateninnervall, das auf das Herzrateninnervall des Ankerpunktes folgt) aufgetragen. So wird also für i=1..m vorgegangen. Ebenso wird für die davorliegenden Herzrateninnervalle i=-1...-m vorgegangen. Auf diese Art und Weise werden alle Mittelwerte zu den 15 vor dem Ankerpunkt liegenden Herzrateninnervall und 15 nach dem Ankerpunkt liegenden Herzrateninnervall aufgetragen. Zu beachten ist, dass der BRS-Indikator ein reiner Zeitwert ist, der keinen Blutdruckwert (mmHg) enthält. Der in FIG. 5 angegebene BRS-Wert von 4,612 Millisekunden ergibt sich dann aus:

$$BRS = \frac{1}{4}[\overline{RR}_{(0)} + \overline{RR}_{(1)} - \overline{RR}_{(-1)} - \overline{RR}_{(-2)}]$$

Hierbei handelt es sich um den 1. Koeffizient einer Haar-Wavelet Zerlegung. Die Berechnung des BRS Wertes aus der Kurvendarstellung ist auch mit anderen Methoden (bspw. Steigung um den zentralen Punkt oder Frequenzanalyse) möglich.

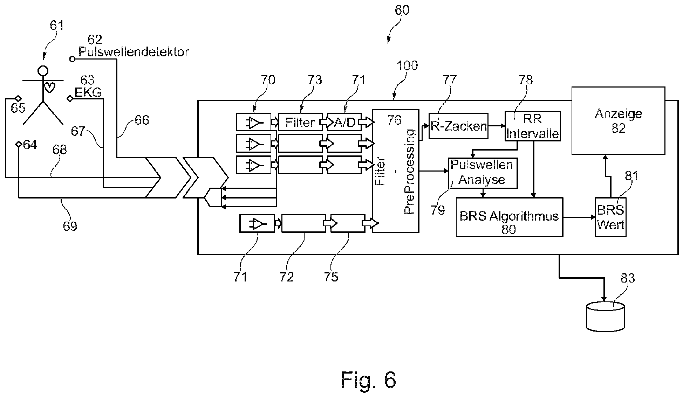

FIG. 6 ist eine weitere vereinfachte Darstellung einer Vorrichtung gemäß der vorliegenden Erfindung. Links befindet sich der Proband 61, dessen EKG und Pulswellenkurve zur Bestimmung beispielsweise von BRS-Werten aufgezeichnet werden. Die Vorrichtung 60 umfasst einen Pulswellensensor bzw. Pulswellendetektor 62, EKG-Elektroden 63, 64, 65 sowie entsprechende Verkabelung 66, 67, 68, 69. Sie werden an die Verarbeitungselektronik 100 angeschlossen. Verarbeitungselektronik 100 umfasst geeignete Vorverstärker 70, 71 zur Vorverstärkung der Signale vom Pulswellensensor 62 und den EKG-Elektroden 63, 64, 65. Die Signale aus den Vorverstärkern werden typischerweise gefiltert in den Filtern 73, 72 und dann in Analog-Digital-Wandlern 74, 75 digitalisiert. Die so digitalisierten Signale werden an einer Verarbeitungseinheit 76 weitergeleitet, in der die Signale typischerweise gefiltert und vorverarbeitet werden. Zum einen werden in der Einheit 76 die R-Zacken des EKG-Signals bestimmt, zum anderen wird eine bereinigte Pulswellenkurve ausgegeben. Aus den R-Zacken werden die Herzrateninnervalle RRi in den Blöcken 76, 77, 78 bestimmt. Basierend auf den Herzrateninnervallen eines jeden Herzschlags werden in der Verarbeitungseinheit 79 zur Pulswellenanalyse die zugehörigen Pulswellenlaufzeiten eines jeden Herzrateninnervalls bestimmt. Die Herzrateninnervalle und die Ergebnisse der Pulswellenanalyse an Verarbeitungsblock 80 weitergegeben, in dem der Algorithmus zur Bestimmung der Baroreflexsensitivität (BRS) angewandt wird. Hieraus ergeben sich die Baroreflexsensitivitätswerte bzw. Indikatoren (BRS-Werte) 81, welche auf einer Anzeige 82 angezeigt und/oder auch in einer Speichervorrichtung 83 gespeichert werden können. Selbstverständlich können die BRS-Werte oder auch andere Werte und Signale weiteren Signalverarbeitungen unterzogen werden.

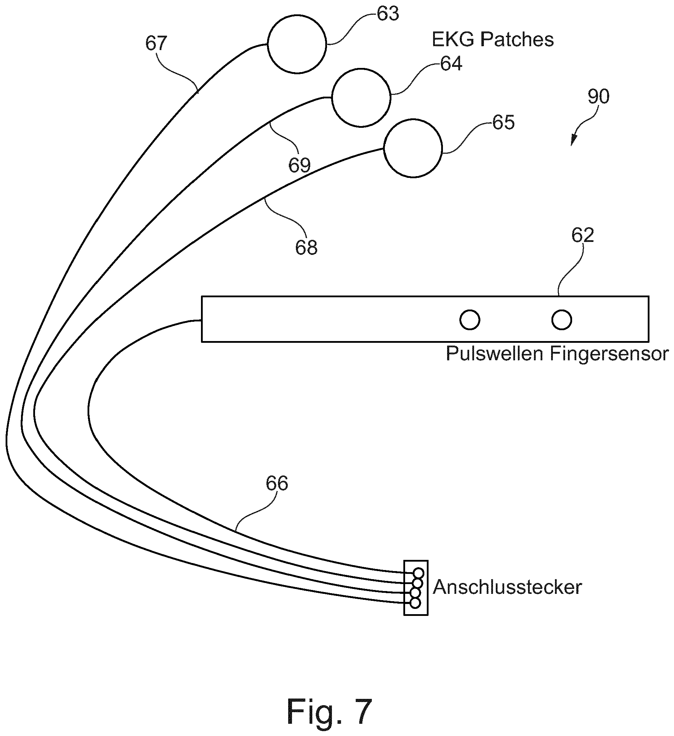

FIG. 7 zeigt ein Ausführungsbeispiel 90, aus dem sich ergibt, dass die EKG-Elektroden 63, 64, 65 mit ihren Anschlussleitungen 67, 69, 68 an einen Anschlussstecker angeschlossen sind, welche ebenfalls die entsprechende Verkabelung zum Pulswellensensor 62 erhält. Ebenso ist der Pulswellensensor 62 dargestellt. In einem bevorzugten Ausführungsbeispiel wird also lediglich ein einziger Anschlussstecker vorgesehen, in dem alle Anschlussleitungen der EKG-Elektroden und des Pulswellensensors 62 mechanisch zusammengeführt sind. Dies kann die Handhabung der Vorrichtung örtlich vereinfachen.

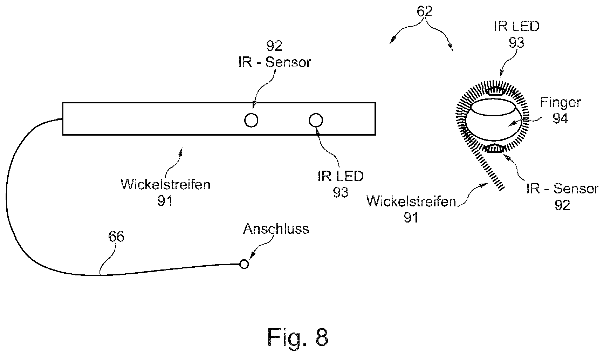

In FIG. 8 ist eine vereinfachte Darstellung des Pulswellensensors 62 zu sehen. Dieser weist einen Wickelstreifen bzw. ein längliches Band oder Pflaster 91 auf. In diesem sind ein IR-Sensor 92 und eine IR-Leuchtiode 93 fest angeordnet. Der Wickelstreifen bzw. der Fingersensor 62 werden zur Anwendung lediglich um den Finger 94 gewickelt. Dabei kommen aufgrund der Abmessungen die IR-Leuchtiode 93 und der IR-Sensor 92 auf gegenüberliegenden Seiten des Fingers 94 zur Anlage.

Insgesamt werden gemäß den Ausführungsbeispielen und Aspekten der Erfindung außerordentlich effiziente, kompakte und kalibrierungsfreie sowie robuste Verfahren, Vorrichtungen und Systeme bereitgestellt, die es erlauben BRS-Indikatoren zu bestimmen.

**Patentansprüche**

1.  Verfahren zur Überwachung und/ oder Messung eines Autoregulationsmechanismus des Blutdrucks bei einem Lebewesen unter Verwendung eines EKG-Signals (31), welches umfasst:

    - Aufzeichnen des EKG-Signals (31) des Lebewesens,
    - Aufzeichnen einer Pulswellenkurve (32) synchron zum Aufzeichnen des EKG Signals (31),

    wobei das Verfahren unter Verwendung eines zum Verarbeiten des EKG-Signals und der Pulswellenkurve eingerichteten Computers (8) ferner die folgenden Schritte umfasst:

    - Bestimmen von Herzratenintervallen aus dem EKG-Signal (31),
    - Bestimmen einer Pulswellenlaufzeit zu jedem Herzratenintervall aus der Pulswellenkurve (32) und dem EKG-Signal (31), insbesondere aus R-Zacken (33) des EKG-Signals (31),
    - Bestimmen einer Mehrzahl signifikanter Änderungen der Pulswellenlaufzeiten nach einem vorgegebenen Kriterium,
    - Auswahl jeweils eines zu jeder signifikanten Änderung der Pulswellenlaufzeiten zeitlich korrelierten Herzrateintervalls als Ankerpunkt,
    - Auswählen einer bestimmten begrenzten Anzahl von zeitlich aufeinanderfolgenden Herzrateintervallen zeitlich vor und/oder nach jedem Ankerpunkt, um so eine begrenzte Sequenz von Herzrateintervallen zu jedem Ankerpunkt zu erzeugen,
    - Mitteln mehrerer korrespondierender Herzrateintervalle jeder Sequenz eines jeweiligen Ankerpunktes über alle Sequenzen der Ankerpunkte,
    - Bestimmen wenigstens eines Baroreflexsensitivitäts-Indikators (81) basierend auf den gemittelten mehreren korrespondierenden Herzrateintervallen.

2.  Verfahren nach Anspruch 1, umfassend: Bestimmen der Herzrateintervalle aus R-Zacken (33) des EKG-Signal (31), wobei ein Herzratenintervall der Zeit zwischen den beiden R-Zacken (33) aufeinanderfolgender Herzschläge entspricht.

3.  Verfahren nach Anspruch 1 oder 2, umfassend: Erkennen von R-Zacken (33) in dem EKG Signal (31) und Bestimmen der Pulswellenlaufzeit eines jeweiligen Herzschlags unter Verwendung der R-Zacke (33) und eines Fußpunktes (34) der Pulswellenkurve (32).

4.  Verfahren nach Anspruch 3, umfassend: Ermitteln des Fußpunktes (34) durch mathematische Anpassung, insbesondere nach einem "Least Squares" Verfahren, eine passende analytische Funktion an den Signalabschnitt ein entsprechender Punkt eines Anstiegs ermittelt werden, der als Fußpunkt (34) dient.

5.  Verfahren nach Anspruch 4, wobei die mathematische Anpassung eine Anpassungen an kontinuierliche Polynome von mindestens Grad drei umfasst, oder die mathematische Anpassung die Verwendung zweier stückweiser definierter Polynome unterschiedlichen Grades umfasst, bei denen entweder ein Übergangspunkt zwischen den Polynomen oder ein anderer charakteristischer Punkt den Fußpunkt (34) repräsentiert.

6.  System (1) zur Überwachung und/ oder Diagnose eines Autoregulationsmechanismus des Blutdrucks bei einem Lebewesen basierend auf einem EKG-Signal (31), welches umfasst:

    eine erste Vorrichtung, welche eingerichtet ist um ein EKG-Signal (31) des Lebewesens aufzuzeichnen, und

eine zweite Vorrichtung, welche mindestens einen Pulswellensensor (62) umfasst, und zum Aufnehmen einer Pulswellenkurve (32) eingerichtet ist,

wobei das System (1) ferner eingerichtet ist zum

- Aufzeichnen des EKG-Signals (31) des Lebewesens unter Verwendung der ersten Vorrichtung,
- Aufzeichnen einer Pulswellenkurve (32) unter Verwendung der zweiten Vorrichtung synchron zum Aufzeichnen des EKG Signals (31),

wobei das System einen zum Verarbeiten des EKG-Signals (31) und der Pulswellenkurve (32) eingerichteten Computer (8) umfasst, der ferner eingerichtet ist zum:

- Bestimmen von Herzratenintervallen aus dem EKG-Signal (31),
- Bestimmen einer Pulswellenlaufzeit zu jedem Herzratenintervall aus der Pulswellenkurve (32),
- Bestimmen einer Mehrzahl signifikanter Änderungen der Pulswellenlaufzeiten nach einem vorgegebenen Kriterium,
- Auswahl jeweils eines zu jeder signifikanten Änderung der Pulswellenlaufzeiten zeitlich korrelierten Herzratenintervalls als Ankerpunkt,
- Auswählen einer bestimmten begrenzten Anzahl von zeitlich aufeinanderfolgenden Herzratenintervallen zeitlich vor und/oder nach jedem Ankerpunkt, um so eine begrenzte Sequenz von Herzratenintervallen zu jedem Ankerpunkt zu erzeugen,
- Mitteln mehrerer korrespondierender Herzratenintervalle jeder Sequenz eines jeweiligen Ankerpunktes über alle Sequenzen der Ankerpunkte
- Bestimmen wenigstens eines Baroreflexsensitivitäts-Indikators (81) basierend auf den gemittelten mehreren korrespondierenden Herzratenintervallen.

7. System (1) nach Anspruch 6, wobei die erste Vorrichtung zur Bestimmung des Herzratenintervalls ein EKG System ist, welches mindestens zwei Elektroden umfasst.

8. System (1) nach einem der vorstehenden Ansprüche 6 oder 7, wobei das EKG System lediglich zur Bestimmung der R-Zacken (33) des EKG Signals (31) ausgestaltet ist und die Herzratenintervalle basierend auf den R-Zacken (33) bestimmt werden.

9. System (1) nach einem der vorstehenden Ansprüche 6 bis 8, wobei die zweite Vorrichtung zur Bestimmung der Pulswellenlaufzeit einen IR-Sensor (92) und eine IR-Leuchtdiode (93) umfasst.

10. System (1) nach Anspruch 9, wobei die IR-Leuchtdiode (93) ausgestattet ist, um bei einer Wellenlänge von 790 nm bis 810 nm, inbesondere 800 nm Licht auszusenden.

11. System (1) nach einem der vorstehenden Ansprüche 9 oder 10, wobei die IR-Diode (93) und der IR-Sensor (92) in einem Fingerclip oder einem Pflaster (91) für einen Finger oder einem (Klett-)Band für einen Finger untergebracht sind.

12. System (1) nach Anspruch 11, wobei der Fingerclip auch eine EKG-Elektrode (63) umfasst.

13. System (1) nach einem der Ansprüche 6 bis 12, wobei das System (1) ferner umfasst: einen ersten Vorverstärker (4), welcher ausgestaltet ist, das Signal des Pulswellensensors (62) zu verstärken und/oder einen zweiten Vorverstärker (5), welcher ausgestaltet ist um Signale von EKG Elektroden (63) zu verstärken und/oder einen Vorfilter (6) zur Vorfilterung der Signale von dem Pulswellensensor (62) und den EKG-Elektroden (63) und/oder einen Analog-Digital-Wandler (74) zur Analog-Digital Wandlung der Signale von dem Pulswellensensor (62) und den EKG-Elektroden (63).

**Claims**

1. A method of monitoring and/or measuring an autoregulation mechanism of the blood pressure in a living being using an ECG signal (31), comprising:

- recording the ECG signal (31) of the living being,
- recording a pulse wave curve (32) synchronously with the recording of the ECG signal (31),

the method further comprising the following steps using a computer (8) arranged to process the ECG signal and the pulse wave curve:

- determining heart rate intervals from the ECG signal (31),
- determining a pulse wave transit time for each heart rate interval from the pulse wave curve (32) and the ECG signal (31), in particular from R waves (33) of the ECG signal (31),
- determining a plurality of significant changes in the pulse wave transit times according to a specified criterion,
- selecting one respective heart rate interval correlated in time with each significant change in the pulse wave transit times as an anchor point,

- selecting a determined limited number of temporally successive heart rate intervals temporally before and/or after each anchor point to thus generate a limited sequence of heart rate intervals for each anchor point,
- averaging a plurality of corresponding heart rate intervals of each sequence of a respective anchor point over all sequences of the anchor points,
- determining at least one baroreflex sensitivity indicator (81) based on the averaged plurality of corresponding heart rate intervals.

2. The method according to claim 1, comprising: determining the heart rate intervals from R waves (33) of the ECG signal (31), a heart rate interval corresponding to the time between the two R waves (33) of successive heartbeats.

3. The method according to claim 1 or 2, comprising: detecting R waves (33) in the ECG signal (31) and determining the pulse wave transit time of a respective heartbeat using the R wave (33) and a foot (34) of the pulse wave curve (32).

4. The method according to claim 3, comprising: defining the foot (34) by means of a mathematical adaptation, in particular in accordance with a least squares method, of a suitable analytical function to the signal portion to define an appropriate point of a slope which serves as a foot (34).

5. The method according to claim 4, wherein the mathematical adaptation comprises an adaptation to continuous polynomials at least of degree three, or the mathematical adaptation comprises the use of two partially defined polynomials of different degrees in which a transition point between the polynomials or a different characteristic point represents the foot (34).

6. A system (1) for the monitoring and/or diagnosis of an autoregulation mechanism of the blood pressure in a living being on the basis of an ECG signal (31), comprising:

    a first device which is arranged to record an ECG signal (31) of the living being, and
    a second device which comprises at least one pulse wave sensor (62) and is arranged to record a pulse wave curve (32),
    the system (1) being further arranged to

      - record the ECG signal (31) of the living being using the first device,
      - record a pulse wave curve (32) using the second device synchronously with the recording of the ECG signal (31),

the system comprising a computer (8) which is arranged to process the ECG signal (31) and the pulse wave curve (32) and is further arranged to:

      - determine heart rate intervals from the ECG signal (31),
      - determine a pulse wave transit time for each heart rate interval from the pulse wave curve (32),
      - determine a plurality of significant changes in the pulse wave transit times according to a specified criterion,
      - select one respective heart rate interval correlated in time with each significant change in the pulse wave transit times as an anchor point,
      - select a determined limited number of temporally successive heart rate intervals temporally before and/or after each anchor point to thus generate a limited sequence of heart rate intervals for each anchor point,
      - average a plurality of corresponding heart rate intervals of each sequence of a respective anchor point over all sequences of the anchor points,
      - determine at least one baroreflex sensitivity indicator (81) based on the averaged plurality of corresponding heart rate intervals.

7. The system (1) according to claim 6, wherein the first device for determining the heart rate interval is an ECG system which comprises at least two electrodes.

8. The system (1) according to any of the preceding claims 6 or 7, wherein the ECG system is merely configured to determine the R waves (33) of the ECG signal (31), and the heart rate intervals are determined on the basis of the R waves (33).

9. The system (1) according to any of the preceding claims 6 to 8, wherein for determining the pulse wave transit time, the second device comprises an IR sensor (92) and an IR light-emitting diode (93).

10. The system (1) according to claim 9, wherein the IR light-emitting diode (93) is equipped to emit light at a wavelength of 790 nm to 810 nm, in particular of 800 nm.

11. The system (1) according to any of the preceding claims 9 or 10, wherein the IR diode (93) and the IR sensor (92) are accommodated in a finger clip or a patch (91) for a finger or a (hook-and-loop) strip for a finger.

12. The system (1) according to claim 11, wherein the finger clip also includes an ECG electrode (63).

13. The system (1) according to any of claims 6 to 12, wherein the system (1) further comprises: a first preamplifier (4) which is configured to amplify the signal of the pulse wave sensor (62), and/or a second preamplifier (5) which is configured to amplify signals of ECG electrodes (63), and/or a prefilter (6) for prefiltering the signals from the pulse wave sensor (62) and the ECG electrodes (63), and/or an analog-to-digital converter (74) for analog-to-digital conversion of the signals from the pulse wave sensor (62) and the ECG electrodes (63).

## Revendications

1. Procédé de surveillance et/ou de mesure d'un mécanisme d'autorégulation de la pression artérielle chez un être vivant en utilisant un signal ECG (31), comprenant :

   - l'enregistrement du signal ECG (31) de l'être vivant,
   - l'enregistrement d'une courbe d'ondes de pouls (32) de manière synchronisée avec l'enregistrement du signal ECG (31),

   le procédé, en utilisant un ordinateur (8) aménagé pour le traitement du signal ECG et de la courbe d'ondes de pouls, comprenant les étapes suivantes:

   - la détermination d'intervalles de fréquence cardiaque à partir du signal ECG (31),
   - la détermination d'un temps de transit d'onde de pouls à chaque intervalle de fréquence cardiaque à partir de la courbe d'ondes de pouls (32) et du signal ECG (31), en particulier à partir d'ondes R (33) du signal ECG (31),
   - la détermination d'une pluralité de changements significatifs des temps de transit d'onde de pouls selon un critère prédéterminé,
   - le choix d'un intervalle de fréquence cardiaque respectif corrélé dans le temps avec chaque changement significatif des temps de transit d'onde de pouls en tant que point d'ancrage,
   - le choix d'un nombre limité déterminé d'intervalles de fréquence cardiaque successifs dans le temps avant et/ou après, dans le temps, chaque point d'ancrage pour ainsi produire une séquence limitée d'intervalles de fréquence cardiaque à chaque point d'ancrage,
   - le moyennage de plusieurs intervalles de fréquence cardiaque correspondants de chaque séquence d'un point d'ancrage respectif sur toutes les séquences des points d'ancrage,
   - la détermination d'au moins un indicateur de sensibilité de baroréflexe (81) sur la base de la moyenne des plusieurs intervalles de fréquence cardiaque correspondants.

2. Procédé selon la revendication 1, comprenant: la détermination des intervalles de fréquence cardiaque à partir d'ondes R (33) du signal ECG (31), un intervalle de fréquence cardiaque correspondant au temps entre les deux ondes R (33) de battements de cœur successifs.

3. Procédé selon la revendication 1 ou 2, comprenant : l'identification d'ondes R (33) dans le signal ECG (31) et la détermination du temps de transit d'onde de pouls d'un battement de cœur respectif en utilisant l'onde R (33) et une base (34) de la courbe d'ondes de pouls (32).

4. Procédé selon la revendication 3, comprenant : la détection de la base (34) par adaptation mathématique, en particulier selon un procédé des moindres carrés, d'une fonction analytique appropriée au tronçon de signal pour déterminer un point correspondant d'une montée qui sert de base (34).

5. Procédé selon la revendication 4, l'adaptation mathématique comprenant une adaptation à des polynômes continus d'un degré d'au moins trois, ou l'adaptation mathématique comprenant l'utilisation de deux polynômes partiellement définis de degré différent, dans lesquels soit un point de transition entre les polynômes, soit un autre point caractéristique représente la base (34).

6. Système (1) de surveillance et/ou de diagnostic d'un mécanisme d'autorégulation de la pression artérielle chez un être vivant sur la base d'un signal ECG (31), comprenant :

   un premier dispositif qui est aménagé de manière à enregistrer un signal ECG (31) de l'être vivant, et
   un deuxième dispositif qui comprend au moins un capteur d'onde de pouls (62) et qui est aménagé pour l'enregistrement d'une courbe d'ondes de pouls (32),
   le système (1) étant en outre aménagé pour

   - enregistrer le signal ECG (31) de l'être vivant en utilisant le premier dispositif,
   - enregistrer une courbe d'ondes de pouls (32) en utilisant le deuxième dispositif de manière synchronisée avec l'enregistrement du signal ECG (31),

   le système comprenant un ordinateur (8) aménagé pour le traitement du signal ECG (31) et de la courbe d'ondes de pouls (32), lequel est en outre aménagé pour :

- la détermination d'intervalles de fréquence cardiaque à partir du signal ECG (31),
- la détermination d'un temps de transit d'onde de pouls à chaque intervalle de fréquence cardiaque à partir de la courbe d'ondes de pouls (32),
- la détermination d'une pluralité de changements significatifs des temps de transit d'onde de pouls selon un critère prédéterminé,
- le choix d'un intervalle de fréquence cardiaque respectif corrélé dans le temps avec chaque changement significatif des temps de transit d'onde de pouls en tant que point d'ancrage,
- le choix d'un nombre limité déterminé d'intervalles de fréquence cardiaque successifs dans le temps avant et/ou après, dans le temps, chaque point d'ancrage pour ainsi produire une séquence limitée d'intervalles de fréquence cardiaque à chaque point d'ancrage,
- le moyennage de plusieurs intervalles de fréquence cardiaque correspondants de chaque séquence d'un point d'ancrage respectif sur toutes les séquences des points d'ancrage,
- la détermination d'au moins un indicateur de sensibilité de baroréflexe (81) sur la base de la moyenne des plusieurs intervalles de fréquence cardiaque correspondants.

7. Système (1) selon la revendication 6, le premier dispositif pour la détermination de l'intervalle de fréquence cardiaque étant un système ECG qui comprend au moins deux électrodes.

8. Système (1) selon l'une des revendications 6 ou 7, le système ECG étant uniquement réalisé pour la détermination des ondes R (33) du signal ECG (31), et les intervalles de fréquence cardiaque étant déterminés sur la base des ondes R (33).

9. Système (1) selon l'une des revendications 6 à 8, le deuxième dispositif pour la détermination du temps de transit d'onde de pouls comprenant un capteur IR (92) et une diode électroluminescente IR (93).

10. Système (1) selon la revendication 9, la diode électroluminescente IR (93) étant équipée de manière à émettre de la lumière à une longueur d'onde de 790 nm à 810 nm, en particulier de 800 nm.

11. Système (1) selon l'une des revendications précédentes 9 ou 10, la diode IR (93) et le capteur IR (92) étant logés dans un clip pour doigt ou dans un pansement (91) pour un doigt ou dans une bande (velcro) pour un doigt.

12. Système (1) selon la revendication 11, le clip pour doigt comprenant également une électrode ECG (63).

13. Système (1) selon l'une des revendications 6 à 12, le système (1) comprenant en outre : un premier préamplificateur (4) qui est réalisé de manière à amplifier le signal du capteur d'onde de pouls (62) et/ou un deuxième préamplificateur (5) qui est réalisé de manière à amplifier des signaux d'électrodes ECG (63) et/ou un préfiltre (6) pour préfiltrer les signaux du capteur d'onde de pouls (62) et des électrodes ECG (63) et/ou un convertisseur analogique-numérique (74) pour la conversion analogique-numérique des signaux du capteur d'onde de pouls (62) et des électrodes ECG (63).

10

1

Pulswellen-Sensor
2

EKG Elektroden
3

Vorverstärker
4

Vorverstärker
5

ADC
Vorfilter
6

7

8

# Fig. 1

```
┌─────────────────────┐              ┌─────────────────────┐
│   Digitale Daten     │              │   Digitale Daten     │
│       EKG            │              │     Oulswelle        │
│       11             │              │       15             │
└─────────────────────┘              └─────────────────────┘
          │                                     │
          ▼                                     ▼
┌─────────────────────┐              ┌─────────────────────┐
│ R-Zacken Erkennung  │─────────┐ ┌─▶│Einteilung in Herz-  │
│       12            │         │ │  │     intervalle       │
└─────────────────────┘         │ │  │       16             │
          │                     │ │  └─────────────────────┘
          ▼                     │ │             │
┌─────────────────────┐         │ │             ▼
│ Artefakt-Erkennung  │         │ │  ┌─────────────────────┐
│       13            │         │ │  │ Artefakt-Erkennung  │
└─────────────────────┘         │ │  │  und herausfiltern   │
          │                     │ │  │       17             │
          │                     │ │  └─────────────────────┘
          │                     │ │             │
          │                     │ │             ▼
          │                     │ │  ┌─────────────────────┐
          │                     │ │  │  Fußpunkt Analyse    │
          │                     │ │  │       18             │
          │                     │ │  └─────────────────────┘
          │                     │ │             │
          │                     └─┼───▶         ▼
          │                       │  ┌─────────────────────┐
          │                       │  │ Pulswellenlaufzeit   │
          │                       │  │    Berechnung        │
          │                       │  │       19             │
          ▼                       │  └─────────────────────┘
┌─────────────────────┐           │             │
│ Herzratenintervalle │───────────┘             ▼
│       14            │           ┌─────────────────────┐
└─────────────────────┘           │ Pulswellenlaufzeiten │
          │                       │       20             │
          │                       └─────────────────────┘
          │                                 │
          └──────────────┬──────────────────┘
                         ▼
            ┌─────────────────────────┐
            │      Gemeinsame          │
            │  Pulslwellenlaufzeit     │
            │    and Herzrate          │
            │        21                │
            └─────────────────────────┘
                         │
                         ▼
            ┌─────────────────────────┐
            │        BPRSA             │
            │       Methode            │
            │         22               │
            └─────────────────────────┘
                         │
                         ▼
               ╱─────────────────╲
              ╱      BRS           ╲
             ╱  Indikator-Wert      ╲
            ╱        23              ╲
            ╲────────────────────────╱
```

# Fig. 2

Fig. 3

Fig. 4

Fig. 5

Fig. 6

Fig. 7

92
IR - Sensor

62

IR LED
93

Finger
94

Wickelstreifen
91

IR LED
93

Wickelstreifen
91

IR - Sensor
92

66

Anschluss

Fig. 8

**IN DER BESCHREIBUNG AUFGEFÜHRTE DOKUMENTE**

**In der Beschreibung aufgeführte Patentdokumente**

- WO 2005043628 A2

- US 7163512 B1

**In der Beschreibung aufgeführte Nicht-Patentliteratur**

- Bivariate phase-rectified signal averaging - a novel technique for cross-correlation analysis in noisy nonstationary signals. *Journal of Electrocardiology,* 2009, vol. 42, 602-606